(19) Europäisches Patentamt
European Patent Office
Office européen des brevets

(11) **EP 3 712 155 A1**

(12) **EUROPEAN PATENT APPLICATION**
published in accordance with Art. 153(4) EPC

(43) Date of publication:
**23.09.2020 Bulletin 2020/39**

(21) Application number: **18891344.6**

(22) Date of filing: **22.11.2018**

(51) Int Cl.:
***C07D 493/00*** *(2006.01)* ***A61K 31/635*** *(2006.01)*
***A61P 35/00*** *(2006.01)* ***A61P 11/00*** *(2006.01)*

(86) International application number:
**PCT/CN2018/116826**

(87) International publication number:
**WO 2019/120033 (27.06.2019 Gazette 2019/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2017 CN 201711367463**

(71) Applicants:
- **Nankai University**
  **Tianjin 300350 (CN)**
- **Uni-Hua (Tianjin) Scientific Industries Pty. Ltd.**
  **Tianjin 300110 (CN)**

(72) Inventors:
- **YANG, Cheng**
  **Tianjin 300350 (CN)**
- **YANG, Guang**
  **Tianjin 300350 (CN)**
- **ZHOU, Honggang**
  **Tianjin 300350 (CN)**
- **SUN, Tao**
  **Tianjin 300350 (CN)**
- **LIU, Shuangwei**
  **Tianjin 300350 (CN)**
- **LIU, Xinhua**
  **Tianjin 300350 (CN)**
- **LIU, Tongtong**
  **Tianjin 300350 (CN)**

(74) Representative: **Sun, Yiming**
**HUASUN Patent- und Rechtsanwälte**
**Friedrichstraße 33**
**80801 München (DE)**

(54) **CRYSTAL FORM OF SESQUITERPENE DERIVATIVE, PREPARATION METHOD THEREFOR AND USE THEREOF**

(57) Provided are a crystal form of a sesquiterpene derivative, a preparation method therefor and the use thereof. In an X-ray powder diffraction pattern of crystal form A, there are characteristic diffraction peaks at positions corresponding to the 2θ diffraction angles of 6.840±0.2, 9.390±0.2, 15.980±0.2, 16.830±0.2, 17.780±0.2, 18.760±0.2, 19.340±0.2, 20.400±0.2, 21.910±0.2, 23.280±0.2, 25.520±0.2, 26.680±0.2, 27.490±0.2, 32.110± 0.2, 32.300±0.2, 37.980±0.2 and 44.230±0.2. The crystal form A has a high degree of crystallinity and is basically non-hygroscopic; in addition, same shows more favorable dissolution kinetics and has a bioavailability that can reach 85.8%.

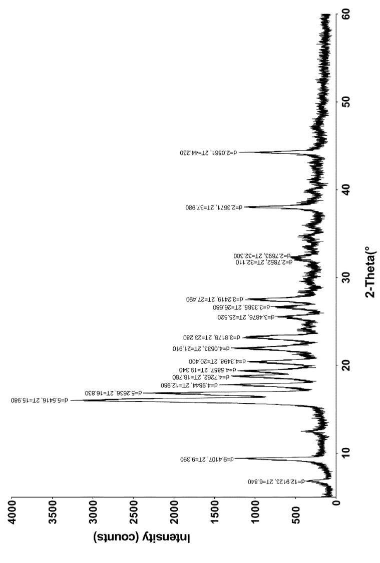

FIG. 1

EP 3 712 155 A1

Printed by Jouve, 75001 PARIS (FR)

## Description

### TECHNICAL FIELD

The disclosure belongs to the technical field of drug crystal forms, and particularly relates to a crystal form of a sesquiterpene derivative, a preparation method therefor and a use thereof.

### DESCRIPTION OF RELATED ART

Sesquiterpene derivatives are used for curing idiopathic pulmonary fibrosis (IPF), which comprise compounds of structural formula (I). In a bleomycin-induced mouse idiopathic pulmonary fibrosis model, the compounds have a certain treatment effect.

In addition, a process for chemically synthesizing the sesquiterpene derivative of structural formula (I) and a use thereof in preparation of anti-tumor drugs have been disclosed in the prior art.

However, the existing sesquiterpene derivative of structural formula (I) is complicated in preparation process, strong in hydroscopicity and prone to hydrolysis or other decomposition processes induced by water. Due to bridge connection between particles by water, active pharmaceutical ingredient (API) particles easily become sticky and are poor in dissolution kinetics performance. Moreover, the existing sesquiterpene derivative of structural formula (I) is low in bioavailability.

### SUMMARY

Aiming at the defects in the prior art, the disclosure provides a crystal form of a sesquiterpene derivative, a preparation method therefor and a use thereof to solve the problems that the existing sesquiterpene derivative is strong in hydroscopicity and prone to hydrolysis or other decomposition processes induced by water; and that due to bridge connection between particles by water, API particles easily become sticky, poor in dissolution kinetics performance and low in bioavailability.

In order to achieve the above object, the technical solution of the disclosure is implemented as follows:
A crystal form A of a sesquiterpene derivative is provided, and a structural formula of the sesquiterpene derivative is as shown in formula (I),

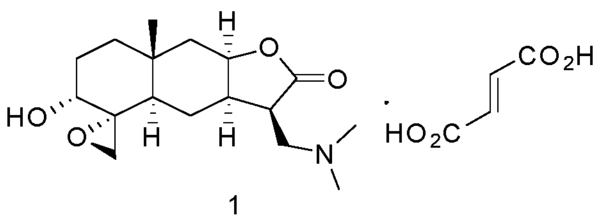

1

(I);

in a X-ray powder diffraction pattern of the crystal form A, there are characteristic diffraction peaks at positions corresponding to 2θ diffraction angles (PXRD) of 6.840 ± 0.2, 9.390 ± 0.2, 15.980 ± 0.2, 16.830 ± 0.2, 17.780 ± 0.2, 18.760 ± 0.2, 19.340 ± 0.2, 20.400 ± 0.2, 21.910 ± 0.2, 23.280 ± 0.2, 25.520 ± 0.2, 26.680 ± 0.2, 27.490 ± 0.2, 32.110 ± 0.2, 32.300 ± 0.2, 37.980 ± 0.2 and 44.230 ± 0.2.

Preferably, in the X-ray powder diffraction pattern of the crystal form A, there are characteristic diffraction peaks at positions corresponding to 2θ diffraction angles of 6.840, 9.390, 15.980, 16.830, 17.780, 18.760, 19.340, 20.400, 21.910, 23.280, 25.520, 26.680, 27.490, 32.110, 32.300, 37.980 and 44.230.

Further, the X-ray powder diffraction pattern of the crystal form A is as shown in Fig. 1.

A method for preparing the crystal form A of the sesquiterpene derivative is further provided, and the crystal form A is prepared by dissolving a compound of structural formula (I) with a single solvent and recrystallizing.

Preferably, the solvent is ethyl acetate.

Preferably, an amount of the ethyl acetate is 10 to 100 times that of the compound of structural formula (I).

Preferably, the heating temperature is 20°C to 80°C.

Preferably, ethyl acetate is used as the solvent, and the amount of the solvent is 25 times that of the compound of structural formula (I); and the heating temperature is 78°C.

A use of the crystal form A of the sesquiterpene derivative is further provided, and the crystal form A of the sesquiterpene derivative is used as a drug.

Preferably, the crystal form A of the sesquiterpene derivative is used as a drug against pulmonary fibrosis.

Preferably, the crystal form A of the sesquiterpene derivative is used as an antitumor drug.

A pharmaceutical composition is further provided, comprising the crystal form A of the sesquiterpene derivative according to any one of claims 1-3.

Further, the pharmaceutical composition comprises one or more pharmaceutically acceptable carriers, excipients or diluents.

Further, the pharmaceutical composition is orally or parenterally administrated. For example, the pharmaceutical composition may be orally administrated, including tablets, capsules, syrups, suspensions or elixir forms that are orally administrated, or compositions suitable for preparing syrups, suspensions or elixir forms in some preferred embodiments. Alternatively, the pharmaceutical composition may be parenterally administrated, including sterile solution or suspension forms that are parenterally administrated, or compositions suitable for preparing sterile solution or suspension in other preferred embodiments.

Preferably, the composition comprises the crystal form A of structural formula (I) serving as an active ingredient, and at least one medicinal adjuvant suitable for inhaling preparations.

Preferably, the pharmaceutical composition is an aerosol inhalant, an aerosol or power aerosols. The pharmaceutical composition is preferably the aerosol.

A recipe of the pharmaceutical composition aerosol is as follows: 1 to 10 weight parts of active ingredient, 5000 to 10000 weight parts of propellant and 100 to 500 parts of solvent. The propellant is selected from one or more of 1,1,1,2-tetrafluoroethane (HFA134a) and 1,1,1,2,3,3,3-heptafluoropropane, and the solvent is selected from one or more of glycerinum, propanediol, polyethylene glycol, ethanol and oleic acid. The propellant is preferably 1,1,1,2-tetrafluoroethane. The preferred solvent is ethanol.

Further, the composition is in a unit dosage form containing 1 mg to1000 mg of the crystal form A of the sesquiterpene derivative.

The pharmaceutical composition is provided for treating various cancers, including gynecological cancers, such as ovarian cancer, cervical cancer, vaginal cancer, pudendum cancer, uterus/endometrial cancer, gestational trophocyte tumor, fallopian cancer and uterine sarcoma; endocrine cancers, such as adrenocortical cancer, pituitary cancer, pancreatic cancer, thyroid cancer, parathyroid cancer, thymic cancer and multiple endocrine tumor; bone cancers, such as osteosarcoma, Ewing's sarcoma and chondrosarcoma; lung cancers, such as small cell lung cancer and non-small cell lung cancer; brain and CNS tumors, such as neuroblastoma, acoustic neuroma, neuroglioma and other brain tumors, spinal cord tumor, breast cancer, colorectal cancer and advanced colorectal adenocarcinoma; gastrointestinal cancers, such as liver cancer, extrahepatic cholangiocarcinoma, gastrointestinal carcinoid tumor, gallbladder cancer, gastric cancer, esophageal cancer and small intestine cancer; urogenital cancers, such as penile cancer, testicular and prostate cancer; head and neck tumors, such as nasal cancer, sinus cancer, nasopharyngeal cancer, oral carcinoma, lip cancer, salivary gland cancer, larynx carcinoma, hypopharyngeal carcinoma and orthopharyngeal cancer; leukemia, such as acute myelogenous leukemia, acute lymphoid leukemia, childhood leukemia, chronic lymphoid leukemia, chronic myeloid leukemia, hairy cellular leukemia, acute promyelocytic leukemia and plasma cellular leukemia; hematologic diseases of bone marrow cancer, such as poor myeloid differentiation syndrome, myeloproliferative diseases, Fanconi anemia, aplastic anemia and idiopathic macroglobulinemia; lymphoid cancers, such as Hodgkin's disease, non-Hodgkin's lymphoma, peripheral T-cell lymphoma, cutaneous T-cell lymphoma and AIDS related lymphoma; eye cancers, including retinoblastoma and uveal melanoma; skin cancers, such as melanoma, non-melanoma skin cancer and Merkel cell carcinoma; soft tissue sarcoma, such as Kaposi's sarcoma, children's soft tissue sarcoma and adult soft tissue sarcoma; urinary system cancers, such as kidney cancer Wilms tumor, bladder cancer, urethra cancer and metastatic cell carcinoma. The pharmaceutical composition is preferably used for treatment of breast cancer, colorectal cancer and lung cancer.

Compared with the prior art, the crystal form A of the sesquiterpene derivative of the disclosure has the following advantages:

The crystal form A of the compound (I) of the disclosure has a high degree of crystallinity, is basically non-hygroscopic, and can be micronized by jet grinding without any change in crystal form; in addition, the possibility of hydrolysis and other decomposition processes induced by water for the crystal form A is lower; the possibility of partially converting from fumarate to free alkali induced due to the presence of water is lower; the possibility that API particles become sticky due to bridge connection between particles in water is lower; the crystal form A shows more favorable dissolution kinetics. Moreover, the bioavailability of the crystal form A of the compound (I) of the disclosure can reach 85.8%.

## BRIEF DESCRIPTION OF THE DRAWINGS

Fig. 1 shows a X-ray powder diffraction pattern of crystal form A of a sesquiterpene derivative; Intensity (counts) refers to the number of particles received by a detector, 2-theta (° refers to an included angle between an incident

light and a reflected light, the d value in the drawing refers to interplanar spacing, and 2T refers to an included angle between the incident light and the reflected light.

Fig. 2 shows a drug concentration-time curve of a crystal form A of a sesquiterpene derivative administrated by tail intravenous injection (30mpk) ; the abscissa Time (h) refers to time points for blood sampling and detection after administration, and the ordinate ng/mL refers to concentrations of drugs in blood samples detected at different time points after administration.

Fig. 3 shows a drug concentration-time curve of a crystal A of sesquiterpene derivative under oral and intragastric administration (150mpk); the abscissa Time (h) refers to time points for blood sampling and detection after administration, and the ordinate ng/mL refers to concentrations of drugs in blood samples detected at different time points after administration.

Fig. 4 shows results of pathological sections in an animal pharmacological experiment.

Fig. 5 shows a pulmonary fibrosis area in an animal pharmacological experiment; in Fig. 5, fibrotic percentage (a proportion of pulmonary fibrosis area): a percentage of pulmonary fibrosis area to total area.

Fig. 6 shows an expression result of hydroxyproline in an animal pharmacological experiment; hydroxyproline: pulmonary fibrosis exhibits accumulation of collagen, hydroxyproline is a unique amino acid in collagen, and the level of hydroxyproline can be used to evaluate the degree of pulmonary fibrosis.

Fig. 7 shows a forced vital capacity (FVC) in an animal pharmacological experiment; FVC: 30 cm $H_2O$ pressure is given to mice by mechanical assistant ventilation. When the maximum value of lung capacity is reached, expiration is made to the greatest extent and at the fastest speed, the vital capacity reaches the minimum value of lung capacity, and their difference value is the forced vital capacity.

In Figs. 4 to 7, NaCl represents sodium chloride group, namely normal saline group in which 0.9% NaCl aqueous solution is administrated; BLM represents bleomycin group in which bleomycin, a drug widely used to construct a mouse pulmonary fibrosis model, is administrated; CP0116 100mpk represents a group administrated a compound of structural formula (I) with a crystal form A; Nintedanib represents a Nintedanib group in which Nintedanib, a drug for treating idiopathic pulmonary fibrosis currently on the market, is administrated as a positive drug in the present disclosure; Pirfenidone represents a Pirfenidone group in which Pirfenidone, a drug for treating idiopathic pulmonary fibrosis currently on the market, is administrated as a positive drug in the present disclosure.

Unless otherwise defined, technical terms used in the following examples have the same meaning generally understood by those skilled in the art. Test reagents used in the following examples, unless specially stated, are all conventional biochemical reagents; the experiment methods, unless specially stated, are all conventional methods.

The disclosure will be described in detail in combination with examples and drawings. The type and parameter of an analytical instrument used in the following embodiments are: X-ray: Cuka36kV20mA. The compound of structural formula (I) as a comparative example 1 is amorphous powder obtained without recrystallization.

**Example 1**

The compound of structural formula (I) (2 g) was mixed with ethyl acetate (20 ml), heated to 78°C under agitation and then refluxed until all solids were dissolved, and then the obtained mixture was naturally cooled under agitation to separate out white crystals. The while crystals were filtered, washed with cold ethyl acetate, and dried in vacuum at 25°C, so as to obtain 1.6g of crystal form A product.

Powder X-ray diffraction analysis was carried out on the crystal form A product obtained in example 1. A powder X-ray diffraction (PXRD) pattern is shown in Fig. 1.

**Example 2**

The compound of structural formula (I) (2 g) was mixed with ethyl acetate (100 ml), heated to 78°C under agitation and then refluxed until all solids were dissolved, and then the obtained mixture was naturally cooled under agitation to separate out white crystals. The while crystals were filtered, washed with cold ethyl acetate, and dried in vacuum at 25°C, so as to obtain 1.2g of crystal form A product.

### Example 3

The compound of structural formula (I) (2 g) was mixed with ethyl acetate (200 ml), heated to 25°C under agitation and then refluxed until all solids were dissolved, and then the obtained mixture was naturally cooled under agitation to separate out white crystals. The while crystals were filtered, washed with cold ethyl acetate, and dried in vacuum at 25°C, so as to obtain 0.92g of crystal form A product.

The crystal form A products prepared in example 1, example 2 and example 3 were taken and placed in weighing bottles for precision weighing. The caps of the bottles were opened, the bottles were put on an upper part of a drier and placed in a constant temperature and humidity incubator with 25°C and 75% humidity, three parts were subjected to parallel operation, and then taken and weighed in two weeks, one month and two months respectively, and the compound amorphous powder of structural formula (I) was used as comparative example 1. After storage for different times, hygroscopicities are as shown in Table 1. Moisture absorption rate = (weight after moisture absorption - weight before moisture absorption)/weight before moisture absorption × 100%.

**Table 1 moisture absorption rates (%) of different compounds of structural formula (I)**

| | **Moisture absorption rates after 2 weeks** | **Moisture absorption rates after 1 month** | **Moisture absorption rates after 2 months** |
|---|---|---|---|
| Example 1 | 1.4 | 2.7 | 3.2 |
| Example 2 | 1.9 | 3.8 | 3.1 |
| Example 3 | 1.6 | 2.2 | 2.8 |
| Comparative example 1 | 8.1 | 12.7 | 22.6 |

It can be seen from the above table that compared with the amorphous compound of structural formula (I), the hygroscopicity of the crystal form A of the compound of structural formula (I) of the disclosure is obviously reduced.

Both of absorption experiments and animal pharmacological experiments use the crystal form A of the compound of structural formula (I) prepared by the following method:
The compound of structural formula (I) was mixed with ethyl acetate to obtain the crystal form A of the compound of structural formula (I) using the method in example 2.

### I. Absorption experiment

(1) Experiment method

Trial rats were Sprague Dawley rats, 7 rats in total, 1 rat was used as blank control, and blank plasma was taken to be used. 3 rats were administrated via tail intravenous injection (30mpk), and 3 rats were orally and intragastrically administrated (150mpk). According to the set time schedule, blood was collected from orbital vein to put in a blood collection vessel containing heparin sodium, centrifuged for 10min at 4°C and 3500rpm, the supernatant of plasma was taken and put into EP tube, and the content of the compound of structural formula (I) in blood was detected. Bioavailability was calculated according to the following formula: bioavailability F% = (AUCpo/AUCIV) × (dose IV/dose po) × 100%;

Time schedule was set, as shown in Table 2.

**Table 2**

| | | | | | | | | | | | |
|---|---|---|---|---|---|---|---|---|---|---|---|
| Tail intravenous injection | 0 | 2mi n | 5min | 10mi n | 20mi n | 30m i n | 1h | 2h | 4h | 6h | 8h |
| Oral and intragastric administrati on | 0 | 5mi n | 15mi n | 30m i n | 1h | 2h | 4h | 6h | 8h | 12 h | / |

(2) Experiment result

Drug concentration-time curves of tail intravenous injection (30mpk) and oral and intragastric administration (150mpk) are shown in Fig. 2 and Fig. 3 respectively.

Pharmacokinetic parameters is shown in Table 3.

| Table 3 | | | | |
|---|---|---|---|---|
| | $T_{max}$ (h) | $T_{1/2}$(h) | $C_{max}$(ng/mL) | AUC(ng/mL/h) |
| Intravenous injection (30 mpk) | 0.11 | 1.35 | 21333 | 2437190 |
| Oral administration (150 mpk) | 0.67 | 1.91 | 56266 | 10452710 |

$$F\% = (AUCpo/AUCIV) \times (dose\ IV/dose\ po) \times 100\%$$

$$= (10452710/2437190) \times (30/150) \times 100\%$$

$$= 85.8\%$$

AUCpo is an area under the drug concentration-time curve under administrating oral drug, AUCIV is an area under the drug concentration-time curve under intravenous injecting drug, dose IV is the dose of the injected drug, the dose po is the dose of the oral drug, $T_{max}$ (h) is the time to reach the blood peak, $T_{1/2}$ (h) is the half-life of blood concentration, and $C_{max}$ (ng/mL) is the drug peak concentration of the drug-time curve.

**II. Animal drug effect**

The crystal form A of the compound of structural formula (I) was obtained from the compound of structural formula (1) through the method in example 2 and then applied to a drug effect test under animal intragastric administration.

(1) Test method

Trial mice were C57BL/6 mice, 35 mice in total, 7 mice in each group, including a saline group, a bleomycin group, a Nintedanib group (100mpk), a Pirfenidone group (200mpk) and a group (100mpk) administrating the crystal form A of the compound of structural formula (I). On day 0, bleomycin (2U/kg) was injected using a trachea to establish an IPF model, 0-7 days were inflammation periods of mice, and 7-14 days were fibrosis lasting progressive periods of mice. Administration was conducted on the day 7, materials were taken on the day 14, the lung functions of mice were measured after mice were narcotized, then the left lungs were taken to make pathological sections, and the right lungs were used to measure hydroxyproline.

(2) Test results

1) Pathological section

The results of pathological sections are shown in Fig. 4. The pathological section can clearly reflect the degree of pulmonary fibrosis in mice. From the pathological sections, the crystal form A of the compound of structural formula (I) can obviously reduce the area of pulmonary fibrosis focus and normalize the alveolar structure.

2) Pulmonary fibrosis area

The pulmonary fibrosis areas are shown in Fig. 5. The pulmonary fibrosis areas in administrated groups are all smaller than pulmonary fibrosis areas in Nintedanib and Pirfenidone groups, and the crystal form A of the compound of structural formula (I) is superior to the positive drugs.

3) Expression of hydroxyproline

Pulmonary fibrosis can cause the activation of pulmonary fibroblasts and excessive collagen secretion, and hydroxyproline is a main component of collagen. The level of collagen can be indirectly displayed using the level of hydroxyproline, as shown in Fig. 6, and the group administrating the crystal form A of the compound of structural formula

(I) can significantly reduce the content of collagen in the lungs.

4) Forced vital capacity

As shown in Fig. 7, the forced vital capacity (FVC) of IPF patients is significantly reduced in clinic, and the crystal form A of the compound of structural formula (I) can significantly improve the FVC of mice.

The crystal form A of the compound of structural formula (I) can significantly alleviate idiopathic pulmonary fibrosis, and is superior to the positive drugs, Pirfenidone and Nintedanib.

In addition, the bioavailabilities of the crystal form A of the compound of structural formula (I) prepared by the methods in example 1 and example 3 are both more than 85.5, which can significantly alleviate idiopathic pulmonary fibrosis.

The above description is only preferred examples of the disclosure, but does not limit the disclosure. Any modification, equivalent replacement, improvement and the like made within the spirit and principles of the disclosure shall be included in the protective scope of the claims.

## Claims

1. A crystal form A of a sesquiterpene derivative, **characterized in that**: a structural formula of the sesquiterpene derivative is as shown in formula (I),

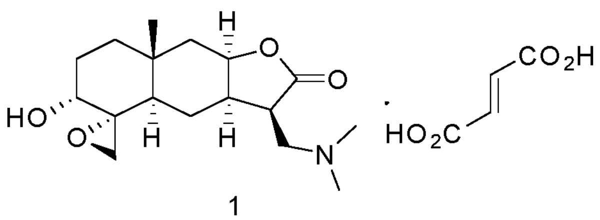

1

(I);

in a X-ray powder diffraction pattern of the crystal form A, there are characteristic diffraction peaks at positions corresponding to 2θ diffraction angles of 6.840 ± 0.2, 9.390 ± 0.2, 15.980 ± 0.2, 16.830 ± 0.2, 17.780 ± 0.2, 18.760 ± 0.2, 19.340 ± 0.2, 20.400 ± 0.2, 21.910 ± 0.2, 23.280 ± 0.2, 25.520 ± 0.2, 26.680 ± 0.2, 27.490 ± 0.2, 32.110 ± 0.2, 32.300 ± 0.2, 37.980 ± 0.2 and 44.230 ± 0.2.

2. The crystal form A of the sesquiterpene derivative according to claim 1, **characterized in that**: in the X-ray powder diffraction pattern of the crystal form A, there are characteristic diffraction peaks at positions corresponding to 2θ diffraction angles of 6.840, 9.390, 15.980, 16.830, 17.780, 18.760, 19.340, 20.400, 21.910, 23.280, 25.520, 26.680, 27.490, 32.110, 32.300, 37.980 and 44.230.

3. The crystal form A of the sesquiterpene derivative according to claim 1, **characterized in that**: the X-ray powder diffraction pattern of the crystal form A is as shown in Fig.1.

4. A method for preparing the crystal form A of the sesquiterpene derivative according to any one of claims 1-3, **characterized in that**: the crystal form A is prepared by dissolving a compound of structural formula (I) with a single solvent and recrystallizing;
preferably, the solvent is ethyl acetate;
preferably, an amount of the ethyl acetate is 10 to 100 times that of the compound of structural formula (I); and preferably, a heating temperature is 20°C to 80°C.

5. The method according to claim 4, **characterized in that**: the ethyl acetate is used as the solvent, and the amount of the solvent is 25 times that of the compound of structural formula (I); and the heating temperature is 78°C.

6. A use of the crystal form A of the sesquiterpene derivative according to any one of claims 1-3, **characterized in that**: the crystal form A of the sesquiterpene derivative is used as a drug;
preferably, the crystal form A of the sesquiterpene derivative is used as a drug against pulmonary fibrosis; and preferably, the crystal form A of the sesquiterpene derivative is used as an antitumor drug.

7. A pharmaceutical composition, **characterized in that**: comprising the crystal form A of the sesquiterpene derivative according to any one of claims 1-3.

8. The pharmaceutical composition according to claim 7, **characterized in that**: further comprising one or more pharmaceutically acceptable carriers, excipients or diluents.

9. The pharmaceutical composition according to claim 7 or 8, **characterized in that**:

   the pharmaceutical composition is orally or parenterally administrated;
   preferably, the composition comprises the crystal form A of structural formula (I) serving as an active component, and at least one medicinal adjuvant suitable for inhaling preparations;
   preferably, the pharmaceutical composition for inhalation is an aerosol inhalant, an aerosol or power aerosols;
   preferably, a recipe of the aerosol is as follows: 1 to 10 weight parts of crystal form A of structural formula (I), 5000 to 10000 weight parts of propellant and 100 to 500 parts of solvent;
   preferably, the propellant is selected from one or more of 1,1,1,2-tetrafluoroethane and 1,1,1,2,3,3,3-heptafluoropropane, and the solvent is selected from one or more of glycerinum, propanediol, polyethylene glycol, ethanol and oleic acid;
   preferably, the propellant is 1,1,1,2-tetrafluoroethane; and
   preferably, the solvent is ethanol.

10. The pharmaceutical composition according to claim 7 or 8, **characterized in that**: the composition is in a unit dosage form containing 1 mg to 1000 mg of the crystal form A of the sesquiterpene derivative.

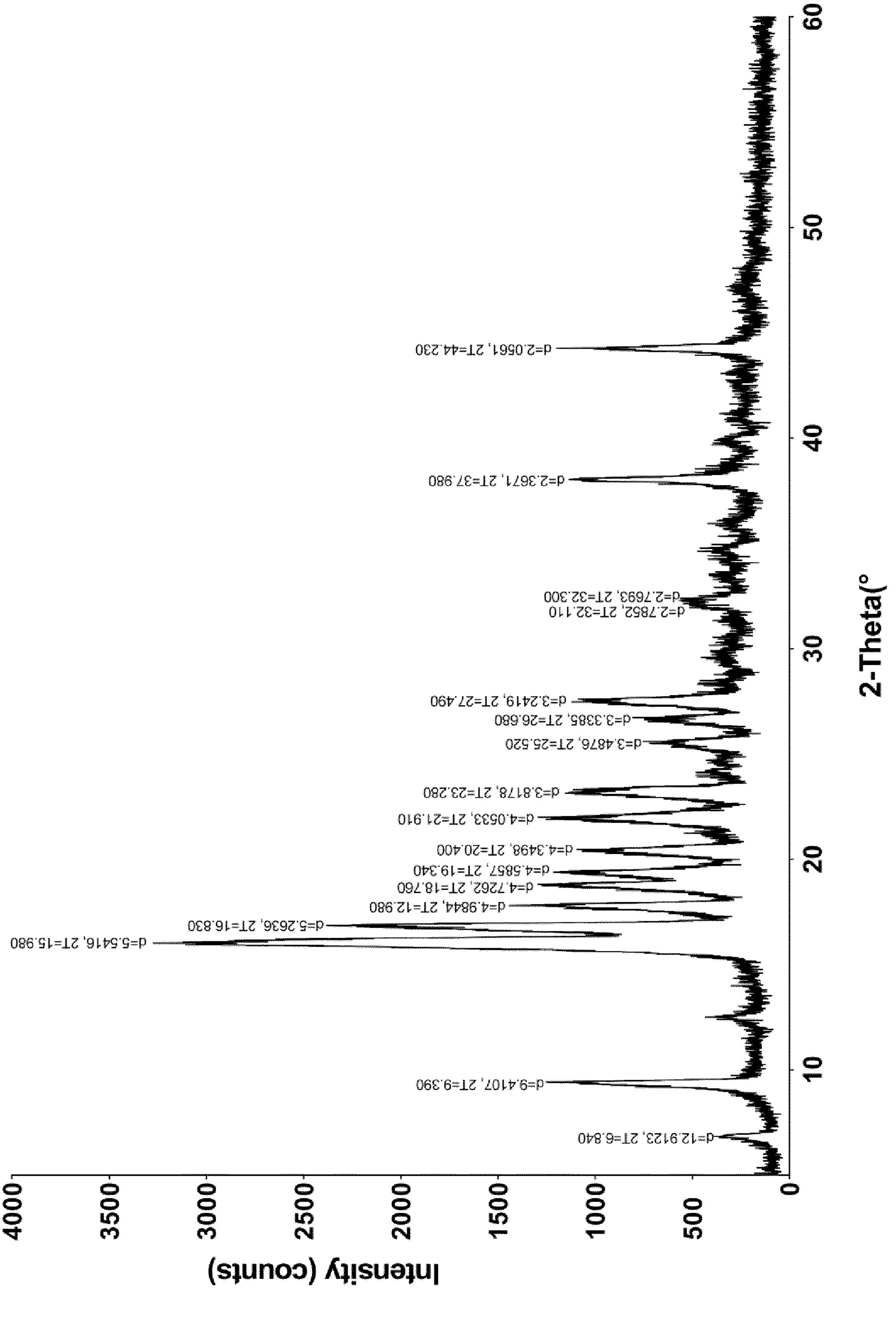
Intensity (counts)
0
500
1000
1500
2000
2500
3000
3500
4000
2-Theta(°
10
20
30
40
50
60

FIG. 1

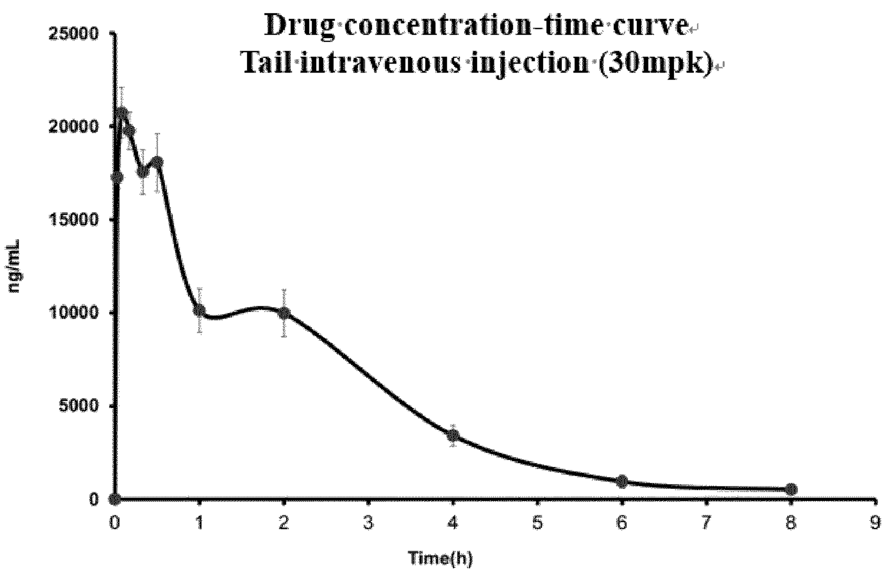
Drug concentration-time curve
Tail intravenous injection (30mpk)
ng/mL
25000
20000
15000
10000
5000
0
0
1
2
3
4
5
6
7
8
9
Time(h)

FIG. 2

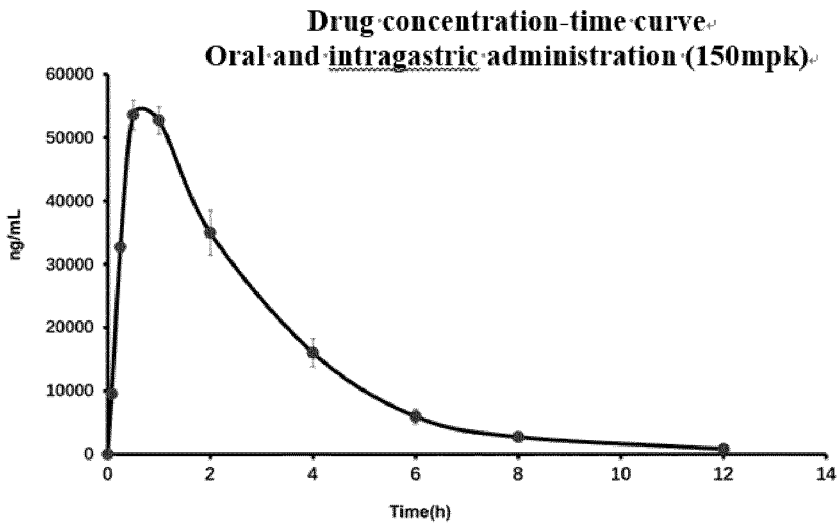
Drug concentration-time curve
Oral and intragastric administration (150mpk)
ng/mL
60000
50000
40000
30000
20000
10000
0
0
2
4
6
8
10
12
14
Time(h)

FIG. 3

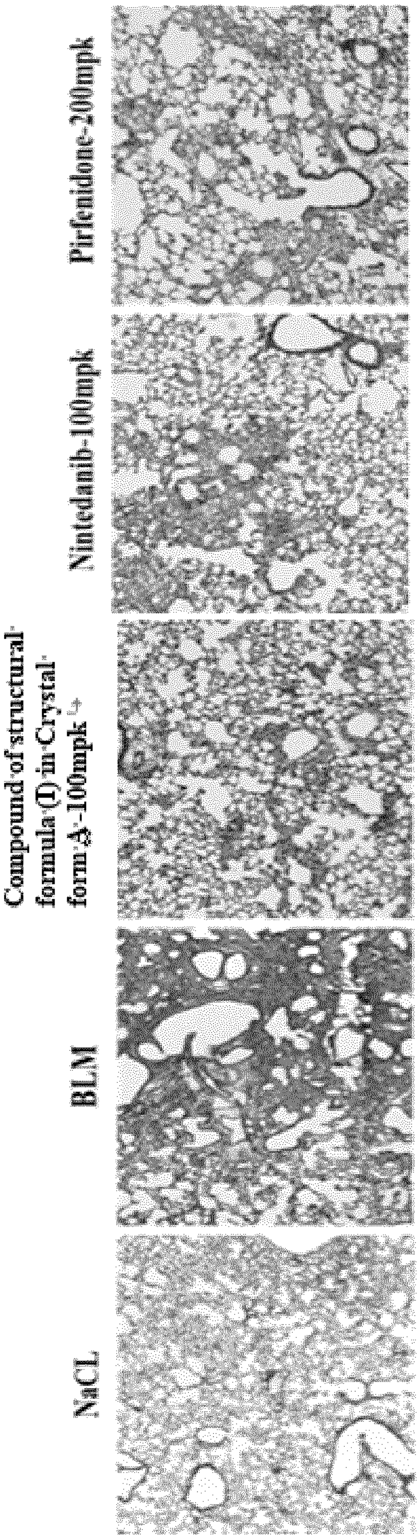
NaCL
BLM
Compound of structural formula (I) in Crystal form A-100mpk
Nintedanib-100mpk
Pirfenidone-200mpk

FIG. 4

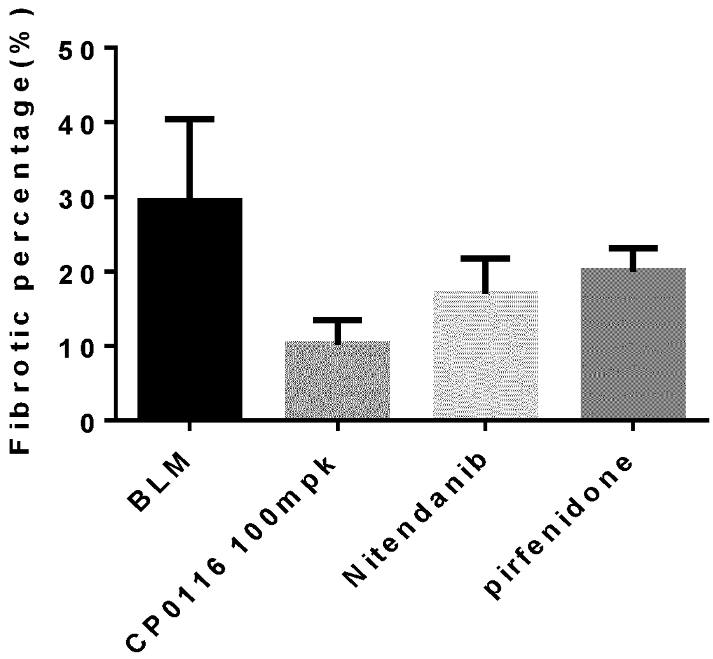
Fibrotic percentage(%)
50
40
30
20
10
0
BLM
CP0116 100mpk
Nitendanib
pirfenidone

FIG. 5

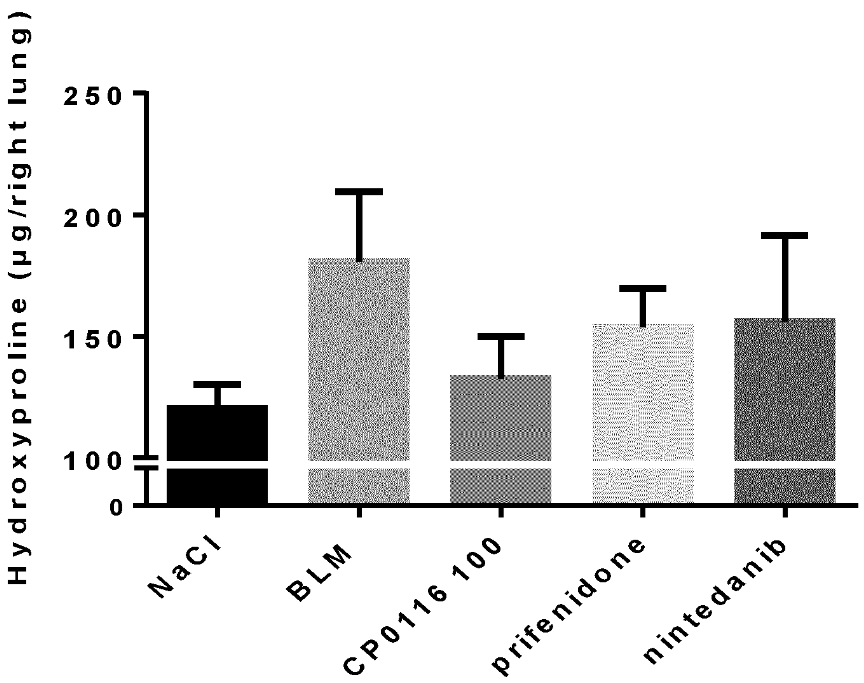
Hydroxyproline (μg/right lung)
250
200
150
100
0
NaCl
BLM
CP0116 100
prifenidone
nintedanib

FIG. 6

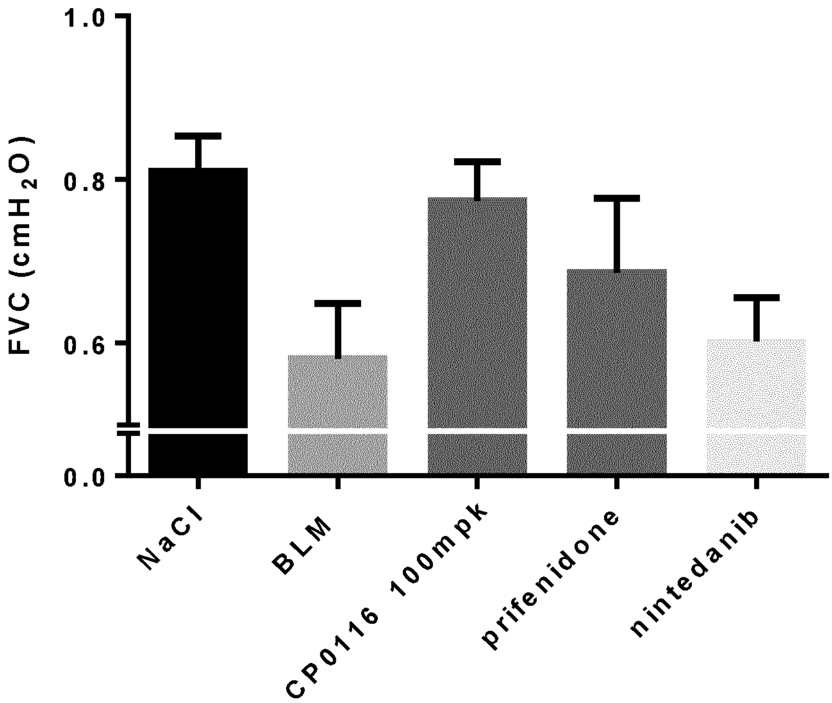

1.0
0.8
0.6
0.0
FVC (cmH2O)
NaCl
BLM
CP0116 100mpk
prifenidone
nintedanib


FIG. 7

**INTERNATIONAL SEARCH REPORT**

International application No. **PCT/CN2018/116826**

**A. CLASSIFICATION OF SUBJECT MATTER**

C07D 493/00(2006.01)i; A61K 31/635(2006.01)i; A61P 35/00(2006.01)i; A61P 11/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT; EPODOC; WPI; CNTXT; USTXT; REGISTRY(STN); CAPLUS(STN): 杨光, 刘新华, 刘双伟, 刘通通, 杨诚, 周红刚, 南开大学, 孙涛, 倍半萜, 晶体, 肺纤维化, 肿瘤, idiopathic, pulmonary, fibrosis, cancer, crystal, sesquiterpene

**C. DOCUMENTS CONSIDERED TO BE RELEVANT**

| Category* | Citation of document, with indication, where appropriate, of the relevant passages | Relevant to claim No. |
|---|---|---|
| X | CN 106496243 A (NANKAI UNIVERSITY) 15 March 2017 (2017-03-15) claims 1-8, and embodiment 3 | 1-10 |
| X | CN 106478569 A (NANKAI UNIVERSITY) 08 March 2017 (2017-03-08) claims 1-10, and embodiment 3 | 1-10 |
| X | CN 106474110 A (NANKAI UNIVERSITY) 08 March 2017 (2017-03-08) claims 1-7, and embodiment 3 | 1-10 |
| X | CN 106474109 A (TIANCHENGNANYUN (TIANJIN) TECH CO., LTD.) 08 March 2017 (2017-03-08) embodiment 3 | 1-10 |
| PX | CN 108003174 A (NANKAI UNIVERSITY) 08 May 2018 (2018-05-08) claims 1-10 | 1-10 |

☐ Further documents are listed in the continuation of Box C. ☑ See patent family annex.

* Special categories of cited documents:

- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 February 2019** | **21 February 2019** |

| Name and mailing address of the ISA/CN | Authorized officer |
|---|---|
| **State Intellectual Property Office of the P. R. China (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2018/116826**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **6**
because they relate to subject matter not required to be searched by this Authority, namely:

[1] The technical solution of claim 6 comprises a method of treating a disease according to the criteria set out in PCT Rule 39.1. This search report is provided in accordance with the technical solution of a use for preparing corresponding pharmaceuticals.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2018/116826**

| Patent document cited in search report | Publication date (day/month/year) | Patent family member(s) | Publication date (day/month/year) |
|---|---|---|---|
| CN 106496243 A | 15 March 2017 | CN 106496243 B | 09 November 2018 |
| CN 106478569 A | 08 March 2017 | None | |
| CN 106474110 A | 08 March 2017 | None | |
| CN 106474109 A | 08 March 2017 | None | |
| CN 108003174 A | 08 May 2018 | None | |

Form PCT/ISA/210 (patent family annex) (January 2015)